(19) Europäisches Patentamt
European Patent Office
Office européen des brevets

(11) **EP 2 548 501 B1**

(12) **EUROPÄISCHE PATENTSCHRIFT**

(45) Veröffentlichungstag und Bekanntmachung des Hinweises auf die Patenterteilung:
**13.03.2019  Patentblatt 2019/11**

(21) Anmeldenummer: **12174252.2**

(22) Anmeldetag: **29.06.2012**

(51) Int Cl.:
*A61B 5/02* *(2006.01)*          *A61B 5/053* *(2006.01)*
*A61B 5/021* *(2006.01)*          *A61B 5/024* *(2006.01)*
*A61B 5/029* *(2006.01)*

(54) **Vorrichtung und Verfahren zur Überwachung physiologischer Signale**

Device and method for monitoring physiological signals

Dispositif et procédé destinés à la surveillance des signaux physiologiques

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.07.2011   US 201161510084 P**

(43) Veröffentlichungstag der Anmeldung:
**23.01.2013   Patentblatt 2013/04**

(73) Patentinhaber: **BIOTRONIK SE & Co. KG**
**12359 Berlin (DE)**

(72) Erfinder:
• **Skerl, Olaf**
**18209 Bad Doberan (DE)**
• **Kirchner, Jens**
**91052 Erlangen (DE)**

(74) Vertreter: **Galander, Marcus et al**
**Biotronik Corporate Services SE**
**Corporate Intellectual Property**
**Sieversufer 7 - 9**
**12359 Berlin (DE)**

(56) Entgegenhaltungen:
**WO-A2-2009/138976      US-A1- 2006 025 699**
**US-A1- 2007 156 057**

**Beschreibung**

Die Erfindung betrifft ein implantierbares Gerät mit einem Positursensor und einem Physiologiesignalsensor. Der Positursensor liefert in seinem implantierten Zustand ein eine Körperlage und/oder eine Änderung einer Körperlage anzeigendes erstes Sensorausgangssignal. Der Physiologiesignalsensor erfasst einen physiologischen Parameter, dessen Wert sich bei einem gesunden Menschen in Reaktion auf eine Änderung der Körperlage (Änderung der Positur, z.B. stehend, liegend, sitzend etc.) ändert. Solch ein Parameter ist beispielsweise der Blutdruck, die intrakardiale Impedanz, das Schlagvolumen, Herztöne, die Herzrate, biochemische Signale und/oder die Sauerstoffkonzentration. Der Physiologiesignalsensor liefert ein zweites Sensorausgangssignal, das den oder die physiologischen Parameter widerspiegelt.

Änderungen der Körperlage führen aufgrund der Schwerkraft zu Umverteilungen des Blutvolumens im Körper. Diese werden durch kurzfristige Kompensationsmechanismen des Herzkreislaufsystems ausgeglichen. Dessen Leistungsfähigkeit und insbesondere der Gesundheitszustand des Herzens zeigen sich im Antwortverhalten der Kompensationsmechanismen.

Geräte, die eine Reaktion eines menschlichen Körpers auf eine Posituränderung erfassen können, sind grundsätzlich bekannt. Bekannte Ansätze sind:

> 1. Messung und Analyse der intrakardialen und intrathorakalen Impedanz
> 2. Bei Blutdruckmessung: systolischer, diastolischer und mittlerer Blutdruck
> 3. Kombination von Blutdruckmessung und Lageänderung: Differenz der mittleren Drücke zwischen zwei Körperlagen

Die vorstehend unter 1. und 2. genannten Lösungen zielen meist auf Änderungen des globalen und statischen Verhaltens der verwendeten Messgröße ab, d.h. solche Änderungen werden detektiert, wenn sie sich in allen Belastungssituationen dauerhaft zeigen.
Gemäß der aus US 2007/0156057 bekannten Lösung werden diejenigen statischen Werte des physiologischen Parameters bei unterschiedlichen Körperlagen erfasst, die sich im Ergebnis bei der jeweiligen Körperlage einstellen (sozusagen "bleibende Regelabweichung").
Bei Verwendung von Blutdrucksignalen ist dazu oft die Verwendung des Relativdrucks nötig, also der gemessene Absolutdruck minus Umgebungsdruck. Damit wird ein zusätzliches Sensorsystem zur Erfassung des Umgebungsdrucks nötig, das weitere Nachteile bieten kann. Ein implantierbares Gerät nach der Präambel von Ansrpuch 1 ist aus US2007156057 bekannt.

Die Erfindung ist in den Ansprüchen 1 und 9 definiert. Ziel der Erfindung ist, ein Gerät zu schaffen, das eine möglichst verbesserte Erkennung von Herzerkrankungen wie CHF (congestive heart failure; kongestives Herzversagen) erlaubt.
Erfindungsgemäß wird zum Erreichen dieses Ziels ein implantierbares Gerät mit einem Positursensor und einem Physiologiesignalsensor vorgeschlagen, von denen

- der Positursensor ausgebildet ist, in seinem implantierten Zustand ein eine Körperlage und/oder eine Änderung einer Körperlage anzeigendes erstes Sensorausgangssignal zu liefern, und
- der Physiologiesignalsensor ausgebildet ist, wenigstens ein wenigstens einen Wert wenigstens eines physiologischen Parameters wie Blutdruck, intrakardiale Impedanz, Schlagvolumen, Herztöne, Herzrate, biochemische Signale und/oder Sauerstoffkonzentration anzeigendes zweites Sensorausgangssignal zu liefern.

Der Positursensor und der Physiologiesignalsensor sind mit einer Auswerteeinheit verbunden, die ausgebildet ist, das erste und das zweite Sensorausgangssignal in Kombination miteinander zu verarbeiten und aus diesen Ausgangssignalen eine oder mehrere Größen zu bestimmen, die das dynamische Verhalten des zweiten Sensorsignals in Reaktion auf eine durch das erste Ausgangssignal angezeigte Körperlageänderung beschreibt.

Die Erfindung schließt den Gedanken ein, statt - wie beim Stand der Technik - lediglich bleibende Regelabweichungen des jeweiligen erfassten physiologischen Parameters in Reaktion auf eine Posituränderung zu erfassen, die Dynamik der Änderung des jeweiligen erfassten physiologischen Parameters in Reaktion auf eine Posituränderung zu erfassen - also einer "Sprungantwort" im regelungstechnischen Sinne vergleichbar.

Die Erfindung schließt die Erkenntnis ein, dass beim Stand der Technik Veränderungen oft erst in einem sehr späten Stadium erkannt werden und dass sich besonders in einem frühen Krankheitsstadium die verminderte Leistungsfähigkeit des Herz-Kreislauf-Systems aber nicht darin zeigt, dass für eine jeweilige Positur typische Werte des jeweiligen physiologischen Parameters nicht mehr erreicht werden, sondern dass es dem Körper mehr "Mühe" macht sie zu erreichen. Der zeitliche Verlauf des physiologischen Signals als Reaktion auf eine Veränderung der Körperlage liefert somit zusätzliche Informationen über Leistungsfähigkeit des Herz-Kreislauf-Systems. Dies wird beim Stand der Technik nicht berücksichtigt.

Das erfindungsgemäße Gerät weist vorzugsweise einen 3-Achs-Akzelerometer, einen Blutdrucksensor und eine Auswerteeinheit auf. Es detektiert geeignete Lageänderungen über den Beschleunigungssensor, bestimmt die Antwort im Blutdrucksignal (sozusagen die "Sprungantwort" des körpereigenen Kompensationsmechanismus) und extrahiert daraus dynamische Parameter des Kompensationsmechanismus. Diese dienen als Maß für die regulatorische Leistungsfähigkeit des Herzens. Sie können zur Detektion und Überwachung von CHF und als Prädiktionsparameter verwendet werden. In der Regelungstechnik heißt diese Methode kurz "Systemidentifikation mittels Sprungantwort".

Ein besonderes Einsatzfeld des erfindungsgemäßen implantierbaren Gerätes ist die Detektion und Überwachung des Krankheitsverlaufs von Herzinsuffizienz (kongestives Herzversagen, congestive heart failure CHF), also das Monitoring von CHF, wobei die Dynamik im Antwortverhalten physiologischer Parameter auf Körperlageänderungen betrachtet wird.

Das erfindungsgemäße Gerät umfasst zumindest einen Sensor und eine Auswerteeinheit, die das folgende Verfahren ausführen:

(a) kontinuierliche Messung einer Größe, die die Körperlage oder deren Änderung erfasst (erstes Sensorausgangssignal);

(b) Detektion einer Änderung der Körperlage, optional Bestimmung des Typs und/oder des Grades dieser Änderung, aus dem ersten Sensorausgangssignal;

(c) kontinuierliche oder zeitweise Messung zumindest eines der folgenden physiologischen Signale: Blutdruck, intrakardialer Impedanz, Schlagvolumen, Herztöne, Herzrate, biochemischer Signale, Sauerstoffkonzentration als zweites Sensorausgangssignal;

(d) vorzugsweise Triggerung der Messung des zweiten Sensorausgangssignals oder Auswahl eines geeigneten Zeitabschnitts aufgrund der Detektion der Lageänderung aus dem ersten Sensorsignal; und

(e) Berechnung eines oder mehrerer Größen, die das dynamische Verhalten der Reaktion des zweiten Sensorsignals an die neue Körperlage beschreibt (z.B. Anstiege, Zeitkonstanten oder Verzögerungszeiten); optional Einbeziehung des Typs/Grades der Lageänderung und/oder weiterer Sensorgrößen.

Bevorzugt wird die Erfassung der Patientenlage mit einem 3D-Beschleunigungssensor realisiert. Das Erkennen der Patientenlage und besonders das Erkennen von deren Änderung können alternativ auch aus dem zweiten Sensorausgangssignal erfolgen, so dass der Positursensor und der Physiologiesignalsensor als ein Sensor verwirklicht sein können.

Vorzugsweise ist die Auswerteeinheit ausgebildet, das Antwortsignal in Teilintervalle zu unterteilen und einzelne Teilintervalle weiter auszuwerten.

So kann die Auswerteinheit beispielsweise ausgebildet sein, das oben genannte Verfahren auszuführen, wobei im Schritt (e) das in Schritt (d) bestimmte Zeitintervall in mehrere Teilintervalle unterteilt wird. Die Ableitung der Größen, die die Dynamik der Kompensation beschreiben, erfolgt vorzugsweise in zumindest einem der Teilintervalle.

Hierbei wird vorzugsweise zumindest eine der folgenden Größen bestimmt:

- Verzögerungszeiten, z.B. die Zeit zwischen der Lageänderung und dem Beginn der Kompensation

- Steigungen des Signals zu bestimmten Zeitpunkten oder in bestimmten Zeitabschnitten (z.B. Druckabfall/Zeit), dabei können über den Zeitabschnitt gemittelte Werte oder Extremwerte bestimmt werden

- Dauer charakteristischer Teilintervalle des Signalverlaufes

- Dauer, die das Signal in einem gegebenen Zeitabschnitt benötigt, um eine gegebene relative Signaldifferenz zu durchlaufen (d.h. die Näherungsbestimmung einer Zeitkonstante)

Bevorzugt sind ein Gerät und ein Verfahren der zuvor geschilderten Art, wobei Schritt (e) zusätzlich den Fit einer Modellfunktion an zumindest einen Abschnitt des Signals umfasst. Die in Schritt (e) bestimmten Größen sind dann geeignete Parameter dieser Modellfunktion.

Für das dynamische Verhalten der Reaktion des zweiten Sensorsignals auf die neue Körperlage kann als Modellfunktion beispielsweise eine Exponentialfunktion zugrunde gelegt werden, z.B.

$$\frac{P(t)}{\Delta P} = 1 - e^{-\frac{t}{\tau}}$$

wobei $\Delta P$ die Veränderung der physiologischen Größe durch die Lageänderung beschreibt. Als geeigneter Parameter der Modellfunktion wird beispielsweise die Zeitkonstante $\tau$ nach bekannten Methoden bestimmt. Eine Veränderung der Zeitkonstante (z.B. Vergrößerung) zeigt sehr frühzeitig eine Veränderung der Leistungsfähigkeit des Kompensationsmechanismus (z.B. Verschlechterung).

Beim Fitten einer gegebenen Modellfunktion ist man an deren Parametern interessiert, beispielsweise an Zeitkonstanten. Daneben ist auch die Form des Antwortverhaltens wichtig, also die Frage, ob z.B. das Signal der Reaktion auf die neue Körperlage linear, exponentiell, sigmoidal, mit einem oder mehreren Überschwingern, oszillierend usw. verläuft. Dazu werden gemäß bevorzugter Ausführungsvarianten verschiedene Modellfunktionen an den Signalabschnitt gefittet und die Güte dieser Fits verglichen. Diagnostisch interessant ist dann, welches der Modelle am besten mit den gemessenen Daten übereinstimmt. Ergänzend können für dieses Modell die Fitparameter betrachtet werden.

Die ermittelten Parameterwerte können als Trend aufgezeichnet oder mit Schwellwerten verglichen werden. Aus den Trends können Trendparameter abgeleitet und ebenfalls mit Schwellwerten verglichen werden. Insbesondere zu diesem Zweck weist das Gerät vorzugsweise einen Speicher auf und ist ebenso vorzugsweise mit einer Telemetrieeinheit ausgestattet, die es erlaubt, Mess- und Parameterwerte z.B. zur weiteren Auswertung zur Analyse durch einen Arzt an ein zentrales Service-Center zu übertragen. Die ermittelten Mess- und Parameterwerte können als Prädiktor zur Vorhersage von Krankheitsereignissen genutzt werden oder von dem Gerät selbst zur Therapiesteuerung und -optimierung eingesetzt werden. Dies ist insbesondere dann vorteilhaft, wenn das Gerät Bestandteil eines implantierbaren Herzstimulators wie beispielsweise eines Herzschrittmachers oder Kardioverters/Defibrillators (ICD) ist.

Die Erfindung soll nun anhand von Ausführungsbeispielen mit Bezug auf die Figuren näher erläutert werden. Die Figuren zeigen Folgendes:

Fig. 1  zeigt als implantierbares medizinisches Gerät einen implantierbaren Herzstimulator 10 und eine daran angeschlossene implantierbare Elektrodenleitung 20.

Fig. 2  zeigt beispielhaft einige Komponenten eines erfindungsgemäßen implantierbaren medizinischen Gerätes, wie es beispielsweise in Figur 1 dargestellt ist.

Fig. 3  zeigt einen schematischen Verlauf des mittleren Blutdrucks P über der Zeit t bei einer Lageänderung.

Fig. 4  zeigt schematische Verläufe des mittleren Blutdrucks P über der Zeit t bei einer Lageänderung.

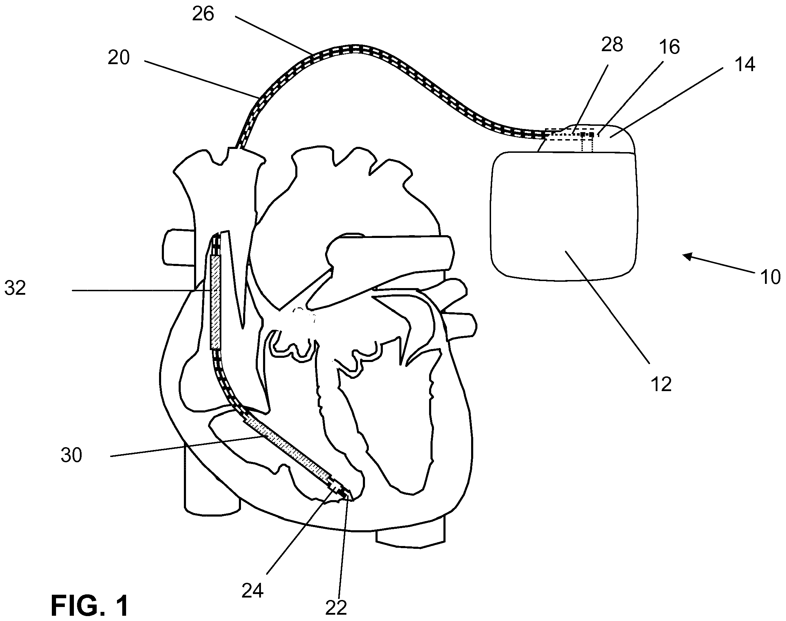

In Figur 1 ist ein implantierbares medizinisches Gerät in Form eines implantierbaren Herzstimulators 10 dargestellt, an den eine Elektrodenleitung 20 angeschlossen ist.

Der implantierbare Herzstimulator 10 kann ein Herzschrittmacher oder ein Kardioverter/Defibrillator (ICD) sein. In dem in Figur 1 dargestellten Ausführungsbeispiel ist der Herzstimulator 10 ein ventrikulärer Herzschrittmacher und Defibrillator. Andere bekannte Herzstimulatoren sind Zweikammerherzschrittmacher zur Stimulation des rechten Atriums und des rechten Ventrikels oder biventrikuläre Herzschrittmacher, die zusätzlich zum rechten Ventrikel auch den linken Ventrikel stimulieren können.

Derartige Stimulatoren besitzen typischerweise ein Gehäuse 12, das im Regelfall aus Metall besteht und somit elektrisch leitend ist und als großflächiger Elektrodenpol dienen kann. An der Außenseite des Gehäuses 12 ist typischerweise ein Anschlussgehäuse 14 befestigt, das auch als Header bezeichnet wird. Ein derartiger Header weist typischerweise Kontaktbuchsen zur Aufnahme von Steckkontakten auf. Die Kontaktbuchsen besitzen elektrische Kontakte 16, die über entsprechende Leiter mit einer in dem Gehäuse 12 des Herzstimulators 10 angeordneten Elektronik verbunden sind.

Die Elektrodenleitung 20 stellt im Sinne dieser Erfindung ein implantierbares medizinisches Gerät mit einem langgestrecktem, elektrischem Funktionsleiter dar. An einem distalen Ende der Elektrodenleitung 20 sind in an sich bekannter Manier Elektrodenpole in Form einer Spitzen- oder Tipelektrode 22 und einer in deren Nähe angeordneten Ringelektrode 24 angeordnet. Die Elektrodenpole 22 und 24 sind so ausgebildet, dass sie je nach Funktion des Herzstimulators, an den die Elektrodenleitung 20 angeschlossen ist, zum Abfühlen elektrischer Potenziale des Herzgewebes (Myokards) dienen oder zur Abgabe elektrischer Signale, beispielsweise zur Abgabe von Stimulationsimpulsen an das sie umgebende Herzgewebe, ausgebildet sind. Fig. 1 zeigt, wie sich die Elektrodenpole, also die Tipelektrode 22 und die Ringelektrode 24, im Anwendungsfall die Elektrodenleitung 20, im Apex des rechten Ventrikels eines Herzens befinden.

Sowohl die Tipelektrode 22 als auch die Ringelektrode 24 sind über jeweils wenigstens einen elektrischen Leiter 26 mit einem Kontakt eines Steckers 28 am proximalen Ende der Elektrodenleitung 20 elektrisch verbunden. Der Stecker 28 besitzt elektrische Kontakte, die mit den elektrischen Kontakten 16 der Kontaktbuchse im Anschlussgehäuse 14 des implantierbaren Herzstimulators korrespondieren.

Die elektrischen Leiter 26 in der Elektrodenleitung 20 können als annähernd gestreckte Seilzugleiter oder als helixförmig gewendelte Leiter ausgebildet sein. Solche Leiter, die funktionale Elektrodenpole mit elektrischen Kontakten des Steckkontaktes am proximalen Ende der Elektrodenleitung 20 elektrisch leitend verbinden, dienen dazu, elektrische Signale von Steckkontakt zum jeweiligen Elektrodenpol zu übertragen oder abgefühlte elektrische Potentiale repräsentierende Signale vom jeweiligen Elektrodenpol zum Steckkontakt zu führen.

Die elektrischen Leiter 26, die die Elektrodenpole 22 bzw. 24 mit den elektrischen Kontakten des Steckers 28 der Elektrodenleitung 20 verbinden, sind über den größten Teil ihrer Länge von einer isolierenden Hülle umgeben, so dass ein elektrischer Kontakt zum Gewebe des Herzens gezielt über die Elektrodenpole zustande kommt.

Neben den Elektrodenpolen 22 und 24, die typischerweise der (in diesem Fall ventrikulären) Stimulation des Herzgewebes dienen, weist die Elektrodenleitung 20 auch noch zwei großflächigere Elektrodenpole 30 und 32 auf, die als Defibrillationselektroden dienen und von wenigstens einem blank liegendem helixartig gewendelten Draht gebildet sind.

Es sei darauf hingewiesen, dass die Erfindung im Rahmen dieses Ausführungsbeispiels anhand eines rechtsventrikulären Herzschrittmachers und Defibrillators erläutert wird. Als medizinisches Gerät im Sinne der Erfindung kann aber jedes andere an sich bekannte elektromedizinische Implantat, also auch ein Mehrkammer-Herzschrittmacher oder Kardioverter/Defibrillator (ICD) oder auch ein Neurostimulator oder ein reines Monitoring-Implantat dienen. Für die Erfindung wesentlich ist, dass das elektromedizinische Implantat neben dem Positursensor mindestens einen Physiologiesignalsensor aufweist, der einen physiologischen Parameter erfasst, dessen Wert sich in Reaktion auf eine Änderung der Körperlage (Änderung der Positur, z.B. stehend, liegend, sitzend etc.) ändert. Solch ein Parameter ist beispielsweise der Blutdruck, die intrakardiale Impedanz, das Schlagvolumen, Herztöne, die Herzrate, biochemische Signale und/oder die Sauerstoffkonzentration. Der Physiologiesignalsensor liefert ein Sensorausgangssignal, das den oder die physiologischen Parameter widerspiegelt.

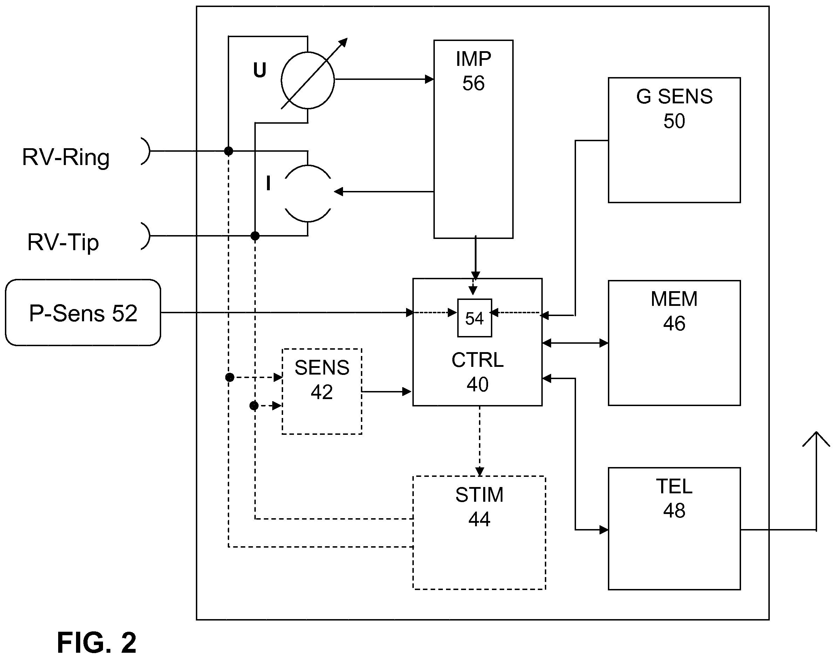

Figur 2 zeigt beispielhaft und schematisch einige Komponenten des Herzstimulators 10 aus Figur 1. Typische Komponenten eines solchen Herzstimulators sind eine Steuereinheit 40, eine oder mehrere Sensingeinheiten 42, die jeweils eine Diagnoseeinheit darstellen und eine oder mehrere Stimulationseinheiten 44, die jeweils eine Therapieeinheit darstellen. Die Steuereinheit 40 ist mit der Sensingeinheit 42 als auch mit der Stimulationseinheit 44 verbunden. Sowohl die Sensingeinheit 42 als auch die Stimulationseinheit 44 sind jeweils mit Elektrodenanschlüssen verbunden, um im Falle der Sensingeinheit 42 elektrische Potentiale des Herzgewebes über die rechtsventrikuläre Ringelektrode 24 und/oder die rechtsventrikuläre Tipelektrode 22 erfassen zu können bzw. im Falle der Stimulationseinheit 44 Stimulationsimpulse beispielsweise über die rechtsventrikuläre Ringelektrode 24 und/oder die rechtsventrikuläre Tipelektrode 22 ausgeben zu können.

Außerdem ist die Steuereinheit 40 mit einer Speichereinheit 46 zum Speichern erfasster Werte von jeweils zu messenden Parametern verbunden. Eine ebenfalls mit der Steuereinheit 40 verbundene Telemetrieeinheit 48 erlaubt es, erfasste Werte von Parametern an ein externes Gerät zu übertragen oder Steuerbefehle von einem externen Gerät zu empfangen.

Die Steuereinheit 40 ist außerdem mit einem 3D-Beschleunigungssensor (3D-Akzeleromter) 50 verbunden, der dazu ausgebildet ist, nicht nur dynamische Beschleunigung z.B. bei körperlicher Aktivität zu erfassen, sondern auch eine jeweilige Gerätelage, die im implantierten Zustand des Gerätes einer jeweiligen Körperlage entspricht. Der 3D-Beschleunigungssensor dient somit als Positursensor.

Außerdem ist die Steuereinheit 40 mit einem Blutdrucksensor 52 verbunden, der bevorzugt dazu ausgebildet ist, den Blutdruck in der Pulmonalarterie zu erfassen und ein entsprechendes Ausgangssignal an die Steuereinheit 40 zu liefern. Der Blutdrucksensor 52 dient als ein Physiologiesignalsensor.

Teil der Steuereinheit 40 ist eine Auswerteeinheit 54, die mit dem Blutdrucksensor 52 und dem 3D-Beschleunigungssensor 50 wenigstens mittelbar verbunden ist und somit die Ausgangssignale dieser Sensoren auswerten kann, wie weiter unten näher erläutert ist.

Die Steuereinheit 40 ist außerdem mit einer Impedanzbestimmungseinheit 56 verbunden. Die Impedanzbestimmungseinheit 56 ist mit einer Stromquelle I und einer Spannungsmesseinheit U verbunden, die ihrerseits wiederum mit den Anschlüssen für die Ringelektrode 24 und die Tipelektrode 22 verbunden sind. Auf diese Weise kann die Gleichstromquelle I konstant Stromimpulse über die Tipelektrode 22 und die Ringelektrode 24 abgeben und die Spannungsmesseinheit U kann die dabei jeweils abfallende Spannung messen. Aus diesen Werten kann die Impedanzbestimmungseinheit 56 einen jeweiligen Impedanzwert bestimmen.

Ein so bestimmter Impedanzwert hängt von verschiedenen Einflussgrößen ab. Beispielsweise würde sich ein Bruch eines elektrischen Leiters in der Elektrodenleitung 20 in einem sehr hohen Impedanzwert äußern. Bei intakter Elektrodenleitung 20 hängt die zwischen den Elektrodenpolen 22 und 24 zu messende Impedanz auch von der Blutmenge im rechten Ventrikel eines Herzens ab, so dass die zu messende Impedanz entsprechend dem Herzzyklus zyklisch schwankt. Beispielsweise steigt die Impedanz mit abnehmendem Blutvolumen, d. h. mit abnehmendem Volumen der rechten Herzkammer, so dass ein zyklischer Anstieg der Impedanz die zyklische Kontraktion der rechten Herzkammer (des rechten Ventrikels) anzeigt. Ebenso kann ein entsprechender Anstieg der gemessenen Impedanz infolge einer Kammerkontraktion nach Abgabe eines Stimulationsimpulses den Stimulationserfolg anzeigen. Auf diese Weise ist die Impedanzbestimmungseinheit 56 in der Lage, eine automatische Stimulationserfolgskontrolle (automatic capture control; ACC) durchzuführen.

Die gemessene Impedanz hängt außerdem von der Impedanz des Elektrodenpol-Gewebekontaktes ab. Daher lässt sich durch Auswerten der gemessenen Impedanzwerte auch eine Ödembildung erfassen, wie sie beispielsweise durch eine Erwärmung der Elektrodenpole infolge magnetischer Wechselfelder eines Kernspintomografen auftreten kann.

Für die vorliegende Erfindung wesentliche Komponenten des implantierbaren medizinischen Gerätes - hier im Beispiel der Herzstimulator 10 - sind ein Lagesensor (z.B. der 3D-Beschleunigungssensor 50) und ein Blutdrucksensor (wie der Blutdrucksensor 52). Der Blutdrucksensor 52 kann gleichzeitig auch als Aktivitätssensor z.B. für die Stimulationsratenanpassung durch die Steuereinheit 40 genutzt werden. In einem alternativen Ausführungsbeispiel ersetzt die Impedanzbestimmungseinheit 56 den Blutdrucksensor 52 als Physiologiesignalsensor.

Beschleunigungssensor 50 und Blutdrucksensor 52 sind - wie in Figur 2 gezeigt - mit der Auswerteeinheit 54 verbunden. Der Beschleunigungssensor 50 und der Blutdrucksensor 52 liefern kontinuierlich oder zyklisch Signale, die den jeweils vom Beschleunigungssensor erfassten Beschleunigungsvektor bzw. Blutdruckwerte widerspiegeln. Die Blutdruckwerte sind vorzugsweise Werte des Blutdrucks in der Pulmonalarterie.

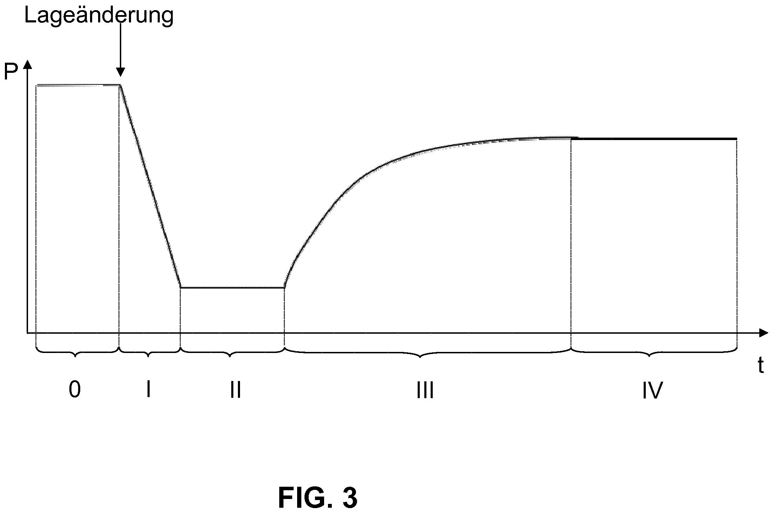

Figur 3 zeigt schematisch den Verlauf eines physiologischen Signals - beispielsweise des Blutdrucks - über der Zeit t bei einer Lageänderung, beispielsweise beim Aufstehen aus einer liegenden in eine stehende Position.

Dabei sind mehrere Phasen zu unterscheiden, deren Dauer in Figur 3 unterhalb der Zeitachse t angegeben ist:

Phase 0. Gleichgewichtsphase vor der Lageänderung

Das physiologische Signal weist einen bestimmten statischen Wert auf.

Phase I. Veränderung des physiologischen Signals aufgrund einer Lageänderung

Dies wird zum Beispiel durch die gravitativ bedingte Umverteilung des Blutvolumens beim Aufstehen verursacht. Das Blut sackt in die Beine, wodurch ein im Rumpfbereich gemessener Druck kurzzeitig abfällt.

Phase II. Verzögerungszeit

Zeitspanne zwischen der Veränderung des physiologischen Signals und dem Einsetzen der Wirkung der körpereigenen Kompensationsmechanismen (regelungstechnisch: "Totzeit")

Phase III. Kompensation

Die körpereigenen Kompensationsmechanismen versuchen, die Abweichung durch die Lageänderung auszuregeln, z.B. damit der Blutdruck wieder ansteigt.

Phase IV. Gleichgewichtsphase nach der Lageänderung

Nach erfolgreicher Regulation schließt sich eine Phase des neuen statischen Zustands an. Der Wert des physiologischen Parameters nach erfolgter Ausregelung kann vom Wert vor der Lageänderung abweichen ("bleibende Regelabweichung").

Bekannte Lösungen bewerten, wie bereits aufgeführt, nur die "bleibende Regelabweichung". Im Unterschied dazu ist die Auswerteinheit 54 zur Bestimmung der dynamischen Parameter des Regelvorgangs (in der Regelungstechnik: Parameter der "Sprungantwort") ausgebildet. Daraus ergeben sich zwei Vorteile: Störungen in den Kompensationsmechanismen des Organismus werden in einem frühen Stadium in den dynamischen Parametern deutlich, lange bevor eine Veränderung in den statischen Parametern ("bleibende Regelabweichung") eintritt. Und für die Bestimmung der dynamischen Parameter sind keine langzeitstabilen oder mit einer Referenz kompensierten Absolutwerte erforderlich.

In Reaktion auf eine Detektion einer Lageänderung aus den Signalen des Lagesensors führt die Auswerteeinheit folgendes durch:

- Bestimmung des Zeitintervalls, das zur Quantifizierung der Kompensationsprozesse analysiert werden soll, insbesondere beginnend nach der gravitativen Blutumverteilung (Blut sackt in die Beine oder strömt in den Oberkörper) bis zum Erreichen eines neuen Gleichgewichtszustands des Blutdrucks.

- Berechnung einer geeigneten Größe für dieses Zeitintervall, die die Dynamik der Kompensation der Blutumverteilung beschreibt, z.B. Steigung des Blutdruckabfalls in Phase I, Zeitdauer bis zum Erreichen der neuen Gleichgewichtslage, Zeitverzögerung bis zum Einsetzen des Kompensationsvorgangs, geeignete Fitparameter (z.B. Zeitkonstante einer exponentiellen Fitfunktion) etc.

Diese Größen liefern eine diagnostische Information über die Leistungsfähigkeit der Kompensationsmechanismen, die beispielsweise mit zunehmender Herzinsuffizienz sinkt.

Für eine Detektion einer Änderung der Körperlage (Änderung der Positur) kommen vorzugsweise folgende Varianten in Frage:
Bevorzugt wird die Änderung der Körperlage mit Hilfe eines 3D-Beschleunigungssensors detektiert.

Alternativ lässt sich die Lageänderung aus einem Blutdruck- oder Blutflusssignal ableiten, in dem plötzliche Änderungen detektiert werden (z.B. mittels Schwellwert für Druckänderung und/oder Test einer Fitfunktion).

Alternativ lässt sich dazu auch ein externes Sensorsystem verwenden, z.B. mittels Kamera. Alternativ können Bett, Stühle etc. mit Drucksensoren ausgestattet sein, die melden, wenn sich der Patient auf den Stuhl setzt, ins Bett legt usw.

Für die Auswertung einer Änderung des Blutdrucks werden die folgenden Varianten bevorzugt:
Bevorzugt wird die Änderung des Blutdrucks nach der gravitativen Umverteilung des Blutvolumens betrachtet (Phasen II & III).

Alternativ kann die Umverteilung selbst betrachtet werden im Zeitintervall von der Lageänderung bis zum Einsetzen der Kompensationsprozesse durch das Herzkreislaufsystem (Phasen I und evtl. II).

Alternativ zur Blutdruckmessung können auch andere Sensorgrößen verwendet werden, z.B. Herzrate oder Herztöne, Blutfluss etc.

Im Folgenden werden Beispiele unterschiedlicher Kurvenverläufe erläutert.

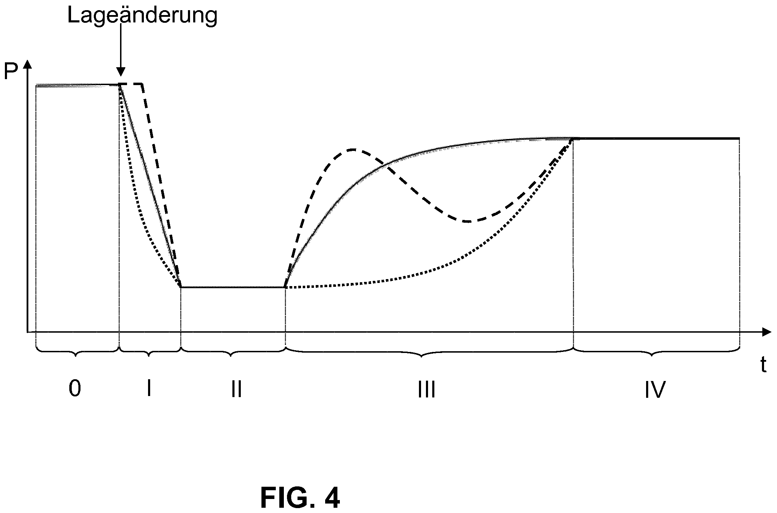

Figur 4 zeigt schematisch mehrere weitere beispielhafte Druckverläufe, die durch gestrichelte und gepunktete Linien im Vergleich zu dem in Figur 3 gezeigten Druckverlauf dargestellt sind. Die Dauer der einzelnen Phasen ist in diesem Beispiel zur Wahrung der Übersichtlichkeit für alle gezeigten Druckverläufe gleich.

■ Verzögerungszeiten

Die Zeitdauer der einzelnen Phasen, insbesondere Phase II und III kann unterschiedlich lang sein. Die Verzögerung, bevor die Kompensationsprozesse einsetzen und das Signal wieder ansteigt (Phase II, sog. "Totzeit") und die Dauer der Kompensation (Phase III) liefern dabei diagnostische Informationen über die regulatorische Leistungsfähigkeit des Organismus. Phase II kann auch fehlen, wenn beispielsweise die Kompensation sofort ohne Verzögerung einsetzt.

■ Unterschiedliche Verläufe

Beim Abfall des Signals in Phase I ist neben einem linearen auch ein exponentieller Verlauf (gepunktete Linie) möglich. Ebenso kann in Phase I eine zusätzliche Verzögerungszeit auftreten bevor die Änderung des physiologischen Parameters sichtbar wird (gestrichelte Linie) Entsprechend können auch in der Kompensationsphase III verschiedene Kurvenformen auftreten (durch die gepunktete und gestrichelten Linien angedeutet): exponentiell, sigmoidal etc.; auch ein stufenweiser Anstieg in mehreren Etappen ist denkbar.

■ Unterschiedliche Geschwindigkeit beim Abfall/Anstieg

Neben der reinen Dauer der einzelnen Phasen und dem Verlauf des physiologischen Signals wird auch das Verhältnis der Werte des physiologischen Signals zu Beginn und Ende der Phasen betrachtet. So kann beispielsweise die Steigung des physiologischen Signals beim Abfall oder Anstieg ermittelt werden.

Gemäß einer bevorzugten Ausführungsvariante führt das Gerät folgende Verfahrensschritte durch:

■ Gleichzeitige kontinuierliche Messung von Blutdruck und Beschleunigung
■ Detektion einer Lageänderung über die Beschleunigung
■ Ab einer detektierten Lageänderung wird, z.B. in einem Intervall von 3 min, der Blutdruckverlauf ausgewertet;
■ Bestimmt wird das Minimum (oder Maximum) der Kurve, um das Ende der gravitativ bedingten Blutumverteilung zu bestimmen;
■ Getestet wird, ob um dieses Minimum herum der Druck eine Zeit lang konstant bleibt. Gegebenenfalls, wird diese Zeitdauer als Verzögerungszeit gespeichert. Sie lässt sich als diagnostisch interessanter Parameter verwenden;
■ An die Teilkurve nach dem Kurvenminimum bzw. am Ende der Verzögerungszeit werden verschiedene Fitfunktionen gefittet, z.B. linearer, exponentieller, sigmoidaler usw. Verlauf. Die Güte dieser Fits (z.B. aus einem $\chi^2$-Wert abgeleitete Werte) werden verglichen und daraus die optimale Fitfunktion bestimmt. Es kann auch a priori eine Modellfunktion angenommen werden, z.B. eine Exponentialfunktion. Diagnostisch interessant sind die Fitfunktion selbst sowie vorrangig die Parameter dieser Funktion, die die Dynamik des Vorgangs beschreiben.

Weitere vorteilhafte Ausführungsvarianten ergeben sich durch die Kombination mit anderen Sensoren.

Die Hinzunahme weiterer Sensoren kann die Spezifität bei der Beurteilung der Kompensationsvorgänge erhöhen. Dadurch können bspw. Belastungssituationen unterschieden werden, die durch einen Aktivitätssensor nicht ausreichend bestimmt werden können. Insbesondere ist zu erwarten, dass eine Lageänderung bei körperlicher Aktivität schneller kompensiert wird als nach einer längeren Ruhephase. Es bietet sich also z.B. an, die Kompensation von Blutdruckschwankungen bei unterschiedlichen Herzraten zu unterscheiden, z.B. in einem Ruhe- und einem Aktivitätsbereich. Analog kann auch der mittlere Blutdruck zur Unterscheidung verschiedener Belastungssituationen verwendet werden.

Die hier vorgestellten Geräte- und Verfahrensvarianten können wie folgt verwendet werden:
Vorzugsweise wird das Gerät bzw. das Verfahren zur Detektion und Überwachung von Herzinsuffizienz (CHF) genutzt. Alternativ lässt sich mit den beschriebenen Methoden auch der Zustand anderer Komponenten der Blutdruckregulation charakterisieren, insbesondere Defekte der Venenklappen. Daneben kann die Wirkung von Medikamenten oder medizinischen Geräten überwacht werden, die solche Regelmechanismen beeinflussen, z.B. Baroreflexstimulatoren.

Neben der Verwendung als Monitor können die berechneten Größen auch zur Adaption des Verhaltens medizinischer Geräte, z.B. eines Schrittmachers oder bei der Dosierung einer (automatischen) Medikamentengabe, verwendet werden.

Die hier vorgestellten Geräte- und Verfahrensaspekte konzentrieren sich auf die Reaktion des Herzkreislaufsystems auf spezifische Belastungsänderungen und erlauben es damit, Änderungen in der Leistungsfähigkeit des Herzens zu detektieren, die sich vorerst nur in diesen Situationen und noch nicht im globalen statischen Verhalten der gemessenen Größe zeigen. Die Methode ist damit sensitiver als Verfahren, die sich beispielsweise auf 24h-Mittelwerte einer Größe stützen.

Ferner werden nicht statische Sollwerte betrachtet, die gerade in der Anfangsphase einer Krankheit noch nicht beeinflusst sind, sondern die dynamischen Parameter von Kompensationsvorgängen des Organismus. Im dynamischen Verhalten werden Änderungen der Leistungsfähigkeit sehr frühzeitig sichtbar, so dass eine höhere Sensitivität als bei anderen Verfahren zu erwarten ist.

Die Belastung ist wohl definiert und wird nicht beeinflusst von der individuellen Tagesgestaltung des Patienten.

Da kurze Abschnitte des Signals und in den meisten Realisierung nur Signaländerungen betrachtet werden, ist eine Kalibration des Signals nicht nötig. Insbesondere können bei dieser Methode Blutdrucksignale verwendet werden, die nicht auf den Umgebungsdruck bezogen sind. Damit entfallen insbesondere die Probleme der Kompensation von Umgebungsdruckänderungen, z.B. durch Wetterumschwung oder Änderung der Höhenposition des Patienten.

## Patentansprüche

1. Implantierbares Gerät mit

   - einem Positursensor, der ausgebildet ist, in seinem implantierten Zustand ein eine Körperlage und/oder eine Änderung einer Körperlage anzeigendes erstes Sensorausgangssignal zu liefern,
   - einem Physiologiesignalsensor, der ausgebildet ist, wenigstens ein wenigstens einen Wert wenigstens eines physiologischen Parameters wie Blutdruck, intrakardialer Impedanz, Schlagvolumen, Herztöne, Herzrate, biochemischer Signale und/oder Sauerstoffkonzentration anzeigendes zweites Sensorausgangssignal zu liefern und
   - einer Auswerteeinheit, die mit dem Positursensor und dem Physiologiesignalsensor verbunden und ausgebildet ist, das erste und das zweite Sensorausgangssignal in Kombination miteinander zu verarbeiten und aus diesen Ausgangssignalen eine oder mehrere Größen zu bestimmen, die das dynamische Verhalten des zweiten Sensorsignals in Reaktion auf eine durch das erste Ausgangssignal angezeigte Körperlageänderung beschreibt,

   **dadurch gekennzeichnet, dass**

   die Auswerteeinheit ausgebildet ist, eine oder mehrere der folgenden Größen zu bestimmen:

   - Die Zeit zwischen einer Änderung der Körperlage und dem Beginn einer eine Kompensation anzeigenden Änderung des zweiten Sensorausgangssignals
   - Dauer eines Teilintervalls des zweiten Sensorausgangssignals
   - Dauer, die das zweite Sensorausgangssignal in einem gegebenen Zeitabschnitt benötigt, um eine gegebene relative Signaldifferenz zu durchlaufen.

2. Implantierbares Gerät gemäß Anspruch 1, **dadurch gekennzeichnet, dass** der Positursensor ein 3D-Beschleunigungssensor ist.

3. Implantierbares Gerät gemäß Anspruch 1 oder 2, **dadurch gekennzeichnet, dass** die Auswerteinheit ausgebildet ist, anhand des ersten Sensorausgangssignals einen Zeitpunkt einer Änderung einer Körperlage zu detektieren.

4. Implantierbares Gerät nach einem der Ansprüche 1 bis 3, **dadurch gekennzeichnet, dass** der Physiologiesignalsensor ein Blutdrucksensor oder ein Impedanzsensor ist.

5. Implantierbares Gerät nach einem der Ansprüche 1 bis 4, **dadurch gekennzeichnet, dass** die Auswerteeinheit ausgebildet ist, das zweite Sensorausgangssignal in mehrere Teilabschnitte zu unterteilen und einzelne Teilintervalle weiter auszuwerten.

6. Implantierbares Gerät nach einem der Ansprüche 1 bis 5, **dadurch gekennzeichnet, dass** die Auswerteeinheit ausgebildet ist, zur Auswertung des zweiten Sensorausgangssignals einen Fit einer Modellfunktion an zumindest einen Abschnitt des zweiten Sensorausgangssignals durchzuführen.

7. Implantierbares Gerät nach und Anspruch 6, **dadurch gekennzeichnet, dass** die Parameter der Modellfunktion von der Auswerteinheit aus dem zweiten Sensorausgangssignal zu bestimmende Größen sind.

8. Implantierbares Gerät nach einem der Ansprüche 1 bis 7, **dadurch gekennzeichnet, dass** das implantierbare Gerät ein Herzstimulator ist.

9. Verfahren zur Herzüberwachung mit den Schritten:

   (a) kontinuierliche Messung einer Größe, die die Körperlage oder dessen Änderung erfasst (erstes Sensorausgangssignal);
   (b) Detektion einer Änderung der Körperlage, optional Bestimmung des Typs und/oder des Grades dieser Änderung, aus dem ersten Sensorausgangssignal;
   (c) kontinuierliche oder zeitweise Messung zumindest eines der folgenden Signale: Blutdruck, intrakardiale Impedanz, Schlagvolumen, Herztöne, Herzrate, biochemische Signale, Sauerstoffkonzentration als zweitem Sensorausgangssignal; und
   (e) Berechnung eines oder mehrerer Größen, die das dynamische Verhalten der Reaktion des zweiten Sensorsignals an die neue Körperlage beschreibt,

**dadurch gekennzeichnet, dass** zumindest eine der folgenden Größen bestimmt wird:

- Zeit zwischen der Änderung der Körperlage und dem Beginn eine eine Kompensation anzeigenden Änderung des ersten Sensorausgangssignals
- Steigungen des ersten Sensorsignals zu bestimmten Zeitpunkten oder in bestimmten Zeitabschnitten
- Dauer charakteristischer Teilintervalle des Signalverlaufes des ersten Sensorsignals,
- Dauer, die das erste Sensorausgangssignal in einem gegebenen Zeitabschnitt benötigt, um eine gegebene relative Signaldifferenz zu durchlaufen.

10. Verfahren nach Anspruch 9, **gekennzeichnet durch** den Verfahrensschritt
(d) Triggerung der Messung des zweiten Sensorausgangssignals oder Auswahl eines geeigneten Zeitabschnitts aus dem zweiten Sensorausgangssignal aufgrund der Detektion einer Änderung der Körperlage aus dem ersten Sensorsignal.

11. Verfahren nach Anspruch 9 oder 10, **dadurch gekennzeichnet, dass** der Verfahrensschritt (e) einen Fit einer Modellfunktion an zumindest einen Abschnitt des zweiten Sensorausgangssignals umfasst.

12. Verfahren nach Anspruch 11, **dadurch gekennzeichnet, dass** die berechneten Größen Parameter der Modellfunktion sind.

13. Verfahren nach Anspruch 11 oder 12, **dadurch gekennzeichnet, dass** die Modellfunktion für wenigstens einen Teilabschnitt des zweiten Sensorausgangssignals eine Exponentialfunktion ist.

**Claims**

1. An implantable device, comprising:

- a posture sensor designed to supply, in the implanted state, a first sensor output signal indicating a body posture and/or a change in a body posture;
- a physiological signal sensor designed to supply at least one second sensor output signal indicating at least one value of at least one physiological parameter such as blood pressure, intracardiac impedance, stroke volume, heart sounds, heart rate, biochemical signals and/or oxygen concentration; and
- an evaluation unit which is connected to the posture sensor and the physiological signal sensor and designed to process the first and second sensor output signals combined with each other and determine, from these output signals, one or more variables that describe the dynamic behavior of the second sensor signal in response to a change in body posture indicated by the first output signal,

**characterized in that**
the evaluation unit is designed to determine one or more of the following variables:

- the time between a change in the body posture and the start of a change of the second output signal that indicates a compensation;
- duration of a sub-interval of the second sensor output signal;
- duration required for the second sensor output signal during a given time segment to pass through a given relative signal difference.

2. The implantable device according to claim 1, **characterized in that** the posture sensor is a 3D acceleration sensor.

3. The implantable device according to claim 1 or 2, **characterized in that** the evaluation unit is designed to detect a time of a change in a body posture based on the first sensor output signal.

4. The implantable device according to any one of claims 1 to 3, **characterized in that** the physiological signal sensor is a blood pressure sensor or an impedance sensor.

5. The implantable device according to any one of claims 1 to 4, **characterized in that** the evaluation unit is designed to divide the second sensor output signal into a plurality of sub-segments and to further evaluate individual sub-intervals.

6. The implantable device according to any one of claims 1 to 5, **characterized in that** the evaluation unit is designed to carry out a fit of a model function to at least one segment of the second sensor output signal in order to evaluate the second sensor output signal.

7. The implantable device according to claim 6, **characterized in that** the parameters of the model function are variables to be determined by the evaluation unit from the second sensor output signal.

8. The implantable device according to any one of claims 1 to 7, **characterized by** being a cardiac stimulator.

9. A method for monitoring the heart, comprising the following steps:

(a) continuously measuring a variable which detects the body posture or the change thereof (first sensor output signal);
(b) detecting a change in the body posture, optionally determining the type and/or the degree of this change, from the first sensor output signal;
(c) continuously or intermittently measuring at least one of the following signals: blood pressure, intracardiac impedance, stroke volume, heart sounds, heart rate, biochemical signals, oxygen concentration, as the second sensor output signal; and
(e) calculating one or more variables which describe the dynamic behavior of the response of the second sensor signal to the new body posture,

**characterized in that** at least one of the following variables is determined:

- time between the change in the body posture and the start of a change of the first output signal that indicates a compensation;
- slopes of the first sensor signal at certain times or in certain time segments;
- duration of characteristic sub-intervals of the signal curve of the first sensor signal;
- duration required for the first sensor output signal during a given time segment to pass through a given relative signal difference.

10. The method according to claim 9, **characterized by** the method step of
(d) triggering the measurement of the second sensor output signal or selection of a suitable time segment from the second sensor output signal based on the detection of a change in the body posture from the first sensor signal.

11. The method according to claim 9 or 10, **characterized in that** the method step (e) comprises a fit of a model function to at least one segment of the second sensor output signal.

12. The method according to claim 11, **characterized in that** the calculated variables are parameters of the model function.

13. The method according to claim 11 or 12, **characterized in that** the model function for at least one sub-segment of the second sensor output signal is an exponential function.

**Revendications**

1. Appareil implantable doté

- d'un capteur de position qui prévu pour donner, dans son état implantable, un premier signal de sortie de capteur indiquant une position du corps et/ou une modification d'une position du corps,
- un capteur de signal physiologique, qui est prévu pour donner au moins une valeur d'au moins un paramètre physiologique comme la tension artérielle, l'impédance intracardiaque, le volume d'éjection systolique, les pulsations cardiaques, les fréquences cardiaques, les signaux biochimiques et/ou un second signal de sortie de capteur indiquant une concentration en oxygène, et
- une unité d'exploitation qui est reliée avec le capteur de position et le capteur de signal physiologique et qui est prévu pour exploiter le premier et le second signal de sortie de capteur en combinaison l'un avec l'autre et pour déterminer, à partir de ces signaux de sortie, une ou plusieurs grandeurs qui décrivent le comportement dynamique du second signal de capteur en réaction à la modification de position du corps indiquée par le premier signal de sortie,

**caractérisé en ce que**
l'unité d'exploitation est prévue pour déterminer une ou plusieurs des grandeurs suivantes :

- le temps entre une modification de la position du corps et le début d'une modification indiquant une compensation du second signal de sortie de capteur,
- la durée d'un intervalle partiel du second signal de sortie de capteur,
- la durée dont a besoin le second signal de sortie de capteur dans un intervalle de temps donné pour délivrer une différence de signal relative donnée.

2. Appareil implantable selon la revendication 1, **caractérisé en ce que** le capteur de position est un accéléromètre tridimensionnel.

3. Appareil implantable selon la revendication 1 ou la revendication 2, **caractérisé en ce que** l'unité d'exploitation est prévue pour détecter l'instant d'une modification d'une position du corps à l'aide du premier signal de sortie de capteur.

4. Appareil implantable selon l'une des revendications 1 à 3, **caractérisé en ce que** le capteur de signal physiologique est un capteur de pression artérielle ou un capteur d'impédance.

5. Appareil implantable selon l'une des revendications 1 à 4, **caractérisé en ce que** l'unité d'exploitation est prévue pour subdiviser le second signal de sortie de capteur en plusieurs segments partiels et pour exploiter ensuite les intervalles partiels individuels.

6. Appareil implantable selon l'une des revendications 1 à 5, **caractérisé en ce que** l'unité d'exploitation est prévue pour effectuer une mise en correspondance d'une fonction modélisée avec au moins un segment du second signal de sortie de capteur pour l'exploitation du second signal de sortie de capteur.

7. Appareil implantable selon la revendication 6, **caractérisé en ce que** les paramètres de la fonction modélisée sont des grandeurs à déterminer par l'unité d'exploitation à partir du second signal de sortie de capteur.

8. Appareil implantable selon l'une des revendications 1 à 7, **caractérisé en ce que** l'appareil implantable est un stimulateur cardiaque.

9. Procédé de surveillance cardiaque avec les étapes :

  (a) de mesure en continu d'une grandeur qui détecte la position du corps ou sa modification (premier signal de sortie de capteur) ;
  (b) de détection d'une modification de la position du corps, éventuellement, de détermination du type et/ou du degré de cette modification à partir du premier signal de sortie de capteur ;
  (c) de mesure en continu ou par intermittence d'au moins un des signaux suivants : pression artérielle, impédance intracardiaque, volume d'éjection systolique, pulsations cardiaques, fréquences cardiaques, signaux biochimiques, concentration en oxygène, en tant que second signal de sortie de capteur ; et
  (e) de calcul d'une ou de plusieurs grandeurs qui décrivent le comportement dynamique de la réaction du second signal de capteur vis-à-vis de la nouvelle position du corps,

  **caractérisé en ce qu'**au moins l'une des grandeurs suivantes est déterminée :

  - le temps entre la modification de la position du corps et le début d'une modification du premier signal de sortie de capteur indiquant une compensation,
  - des pics du premier signal de capteur à des moments déterminés ou pendant des intervalles de temps déterminés,
  - d'intervalles partiels du signal du premier signal de capteur caractérisant la durée,
  - de la durée dont a besoin le premier signal de sortie de capteur en un intervalle de temps donné pour délivrer une différence de signal relative donnée.

10. Procédé selon la revendication 9, **caractérisé par** l'étape de procédé
(d) de déclenchement d'une mesure du second signal de sortie de capteur ou de choix d'un intervalle de temps approprié à partir du second signal de sortie de capteur en raison de la détection d'une modification de la position du corps à partir du premier signal de capteur.

11. Procédé selon la revendication 9 ou la revendication 10, **caractérisé en ce que** l'étape de procédé (e) comprend une mise en correspondance d'une fonction modélisée avec au moins un segment du second signal de sortie de capteur.

12. Procédé selon la revendication 11, **caractérisé en ce que** les grandeurs calculées sont des paramètres de la fonction modélisée.

13. Procédé selon la revendication 11 ou la revendication 12, **caractérisé en ce que** la fonction modélisée pour au moins un segment partiel du second signal de sortie de capteur est une fonction exponentielle.

**FIG. 1**

EP 2 548 501 B1

**FIG. 2**

EP 2 548 501 B1

FIG. 3

**FIG. 4**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20070156057 A
- US 2007156057 A